**Europäisches Patentamt**

**European Patent Office**

**Office européen des brevets**

(19)

(11) Veröffentlichungsnummer: **0 324 360**
**B1**

(12) # EUROPÄISCHE PATENTSCHRIFT

(45) Veröffentlichungstag der Patentschrift:
**30.05.90**

(21) Anmeldenummer: **89100075.4**

(22) Anmeldetag: **04.01.89**

(51) Int. Cl.⁵: **C07C 53/124,** C07C 51/14,
C07C 69/24, C07C 67/38

(54) **Verfahren zur Herstellung von Isobuttersäure oder ihren Derivaten.**

(30) Priorität: **11.01.88 DE 3800466**

(43) Veröffentlichungstag der Anmeldung:
**19.07.89 Patentblatt 89/29**

(45) Bekanntmachung des Hinweises auf die Patenterteilung:
**30.05.90 Patentblatt 90/22**

(84) Benannte Vertragsstaaten:
**AT BE CH DE ES FR GB IT LI NL SE**

(56) Entgegenhaltungen:
**EP-A- 0 031 886**
**US-A- 2 154 795**
**US-A- 4 590 293**

(73) Patentinhaber: **RÖHM GMBH, Kirschenallee,
D-6100 Darmstadt(DE)**

(72) Erfinder: **Ruppert, Wolfgang, Dr.Ing., Weedring 9,
D-6104 Seeheim-Jugenheim(DE)**
Erfinder: **Siegert, Hermann-Josef, Dr.,
Burkhardtstrasse 38, D-6104 Seeheim-Jugenheim(DE)**

## Beschreibung

Die Erfindung betrifft ein Verfahren zur Herstellung von Isobuttersäure oder ihren Derivaten durch Koch'sche Synthese aus etwa stöchiometrischen Mengen an Propylen, Kohlenmonoxid und gegebenenfalls Wasser oder einem Alkohol in flüssigem Fluorwasserstoff als Koch'schem Katalysator. Die zugrundeliegenden Reaktionen laufen schnell und mit beträchtlicher Wärmeentwicklung ab, wenn sie unter hohem Druck und bei hoher Rückvermischung durchgeführt werden. Deshalb ist gerade unter diesen Bedingungen eine intensive Kühlung des Reaktionsgemisches mit einem flüssigen Kühlmittel zur Aufrechterhaltung einer gleichbleibenden Reaktionstemperatur, insbesondere bei einem kontinuierlich betriebenen Verfahren, unerläßlich.

### Stand der Technik

Ein kontinuierlich durchführbares Verfahren zur Herstellung von Isobuttersäure oder ihren niederen Alkylestern auf dem oben angegebenen Weg ist in dem EP-B 31 886 beschrieben. In ähnlicher Weise läßt sich gemäß EP-B 91 604 Isobuttersäurefluorid herstellen. Die Reaktionstemperaturen betragen 100 bis 140 Grad C beim erstgenannten und 40 bis 90 Grad C beim letztgenannten Verfahren. Da die Reaktionen exotherm sind, muß die Reaktionstemperatur durch Kühlung auf einer gleichbleibenden Höhe gehalten werden.

### Aufgabe und Lösung

Zur Aufrechthaltung einer gleichmäßigen Reaktionstemperatur wird dem Reaktionsgemisch an einem Wärmetauscher die überschüssige Reaktionswärme entzogen. Zu diesem Zweck wird der Wärmetauscher mit einem flüssigen Kühlmittel durchströmt. Wasser ist das in der Technik meistgebrauchte Kühlmittel.

Im vorliegenden Fall ist es aus Sicherheitserwägungen ratsam, den hohen Gehalt an Fluorwasserstoff in dem Reaktionsgemisch bei der Wahl des Kühlmittels zu berücksichtigen. Wenn auch aus EP-B 76 367 Werkstoffe bekannt sind, die gegenüber dem Reaktionsgemisch unter den Reaktionsbedingungen beständig sind, darf die Gefahr eines Lecks in der zwischen dem Reaktionsgemisch und dem Kühlmittel gelegenen Wärmetauschfläche nicht außer Betracht bleiben. Die Aufgabe der Erfindung besteht darin, für diesen Fall Schwierigkeiten auszuschließen, die sich aus der Vermischung des Kühlmittels mit dem Reaktionsgemisch ergeben könnten.

Zu den Schwierigkeiten, die erfindungsgemäß ausgeschlossen werden sollen, gehören einerseits die ungewollte Erhitzung des entstehenden Gemisches durch Reaktionen zwischen den Bestandteilen des Reaktionsgemisches mit denen des Kühlmittels, die Bildung von hoch korrosiven Gemischen und die Entwicklung von giftigen Gasen oder Dämpfen. Andererseits kann auch die Notwendigkeit der Entsorgung des entstandenen Gemisches Schwierigkeiten bereiten. Wasser wäre unter diesen Gesichtspunkten ein ungeeignetes Kühlmittel, weil es mit Fluorwasserstoff eine hochkorrosive Mischung bildet. Auch wäßrige Laugen sind unbrauchbar, weil sie unter starker Wärmeentwicklung mit Fluorwasserstoff reagieren würden.

Es wurde nun gefunden, daß die genannte Aufgabe dadurch lösbar ist, daß das Reaktionsgemisch an einem Wärmetauschers auf einer gleichbleibenden Reaktionstemperatur gehalten wird, worin ein Additionsprodukt aus wenigstens zwei der oben genannten Ausgangsstoffe als Kühlmittel in einem geschlossenen Kühlkreislauf verwendet wird. Die Erfindung erstreckt sich auch auf den Fall, daß als Ausgangsstoffe der Isobuttersäure-Herstellung selbst eines oder mehrere der genannten Additionsprodukte eingesetzt wird bzw. werden.

Im Falle eines Lecks gelangen keine Stoffe aus dem Kühlsystem in das Reaktionsgemisch, die dort nicht ohnehin vorhanden sind oder in der gleichen Weise wie die Bestandteile des Reaktionsgemisches reagieren würden. Vorzugsweise liegt der Druck des Kühlmittels unter dem des Reaktionsgemisches, so daß im Falle eines Lecks Teile des Reaktionsgemisches in das Kühlsystem eindringen würden. Dadurch verändert sich zwar dessen Korrosivität und es kommt zu einer Verunreinigung, die nur kurzzeitig bis zur Behebung des Lecks tragbar ist, jedoch treten keine unmittelbaren Folgeschäden ein. Wesentlich ist vor allem, daß das verunreinigte Kühlmittel nicht verloren ist und entsorgt werden muß, sondern daß es in einem Zwischenbehälter aufbewahrt und als Ausgangsstoff für die Herstellung von Isobuttersäure bzw. ihrer Derivate verwendet werden kann. Vorzugsweise geschieht das in begrenzten Anteilen von beispielsweise 1 bis 10% der üblichen Ausgangsstoffe.

### Ausführung der Erfindung

Aus den Ausgangsstoffen, die bei der Koch'schen Synthese von Isobuttersäure oder ihren Derivaten als Reaktanden oder als Katalysator eingesetzt werden, lassen sich verschiedene binäre oder ternäre Additionsprodukte bilden; nämlich

a) Isopropylfluorid aus Propylen und Fluorwasserstoff,

b) Isopropanol und Diisopropyläther aus Propylen und Wasser,

c) Methylisopropyläther aus Propylen und Methanol,

d) Ameisensäure aus Kohlenmonoxid und Wasser,

e) Methylformiat aus Kohlenmonoxid und Methanol,

f) Ameisensäurefluorid aus Kohlenmonoxid und Fluorwasserstoff,

g) Isopropylformiat aus Propylen, Kohlenmonoxid und Wasser.

Unter diesen möglichen Additionsprodukten wird das Kühlmittel so gewählt, daß es in dem Temperaturbereich seiner Anwendung keine merkliche Zersetzung erleidet und keine Korrosion bewirkt. Vorzugsweise wird ein Additionsprodukt mit einem Siedepunkt über 70 Grad C (bei Normaldruck) eingesetzt. Hinsichtlich seiner Beständigkeit bei erhöhter Temperatur, seiner geringen Korrosivität und seines verhältnismäßig niedrigen Dampfdruckes bei der Betriebstemperatur ist Isopropanol in den meisten Fällen das bestgeeignete Kühlmittel, insbesondere bei der Herstellung von Isobuttersäure. Zur Herstellung von Isobuttersäurefluorid ist Isopropylfluorid als Kühlmittel vorzuziehen. Bei der Herstellung von Isobuttersäuremethylester ist Methylisopropyläther verwendbar.

Als Wärmetauscher kann die Wandung des Reaktors, in dem die Umsetzung stattfindet, dienen, wenn sie in an sich bekannter Weise mit einem durchströmbaren Kühlmantel umgeben ist. Man kann auch einen Rohrbündel-Wärmetauscher verwenden, der in das Reaktionsgemisch eingetaucht oder in einem Nebenkreislauf von diesem durchströmt wird.

In Berührung mit der heißen Reaktorwandung wird das Kühlmittel auf eine Temperatur erwärmt, die nahe an der Temperatur des Reaktionsgemisches liegen kann, aber in der Regel 5 bis 20 K darunter bleibt. Vorzugsweise wird Isopropanol auf maximal 140 Grad C erwärmt. Wenn das Kühlsystem auf den Wärmeübergang zwischen zwei flüssigen Medien ausgelegt ist, kann es notwendig sein, das Kühlsystem unter Druck zu betreiben, um zu verhindern, daß das Kühlmittel über den Siedepunkt erhitzt wird; beispielsweise bis zu 10 bar, jedoch soll - wie schon erwähnt - der Druck des Reaktionsgemisches möglichst nicht überschritten werden.

Um die entzogene Wärme an anderer Stelle nutzbar zu machen, kann das Kühlsystem unter einem Druck gehalten werden, bei dem das Kühlmittel an der Wärmetauschwandung verdampft, beispielsweise 0,1 bis 10 bar; die latent aufgenommene Wärme läßt sich bei der Kondensation des Dampfes zurückgewinnen.

Das Kühlmittel wird zweckmäßig mittels einer Umlaufpumpe in einem geschlossenen Kühlkreislauf umgewälzt. Von den Wärmetauschflächen des Reaktors wird es in einen Kühler geleitet, wo es durch ein kühleres flüssiges oder gasförmiges Medium, z.B. Kühlwasser oder -Luft, auf eine niedrigere Temperatur gekühlt oder kondensiert wird. Die Wärme kann auch dazu genutzt werden, einen der Ausgangsstoffe oder ein Prozeßmedium eines verbundenen Verfahrens vorzuwärmen. Von dem Kühler wird es mit einer Temperatur, die unter der Reaktionstemperatur liegt, zum Wärmetauscher des Reaktors zurückgeführt.

Es ist vorteilhaft, das Kühlsystem, insbesondere das Volumen des darin enthaltenen Kühlmittels so klein wie möglich zu halten, damit im Falle eines Lecks nicht zu große Mengen des verunreinigten Kühlmittels zur Wiederverwendung aufbewahrt werden müssen.

## Patentansprüche

1. Verfahren zur Herstellung von Isobuttersäure oder ihren Derivaten durch Koch'sche Synthese aus etwa stöchiometrischen Mengen an Propylen, Kohlenmonoxid und gegebenenfalls Wasser oder einem Alkohol in flüssigem Fluorwasserstoff als Koch'schem Katalysator, dadurch gekennzeichnet, daß das Reaktionsgemisch an einem Wärmetauscher mittels eines Additionsproduktes aus wenigstens zwei der oben genannten Stoffe als Kühlmittel in einem geschlossenen Kühlkreislauf auf einer gleichbleibenden Reaktionstemperatur gehalten wird.

2. Verfahren nach Anspruch 1, dadurch gekennzeichnet, daß das Kühlmittel unter einem geringeren Druck als das Reaktionsgemisch gehalten wird.

3. Verfahren nach Anspruch 2, dadurch gekennzeichnet, daß das Kühlmittel unter einem Druck gehalten wird, bei dem es in dem Wärmetauscher verdampft.

4. Verfahren nach den Ansprüchen 1 bis 3, dadurch gekennzeichnet, daß als Kühlmittel ein Additionsprodukt mit einem Siedepunkt über 70 Grad C (bei Normaldruck) eingesetzt wird.

5. Verfahren nach Anspruch 4, dadurch gekennzeichnet, daß als Kühlmittel Isopropanol eingesetzt wird.

## Claims

1. Process for the preparation of isobutyric acid or derivatives thereof by means of Koch synthesis from approximately stoichiometric amounts of propylene, carbon monoxide and optionally water or an alcohol, in liquid hydrogen fluoride as Koch catalyst, caracterised in that the reaction mixture in a heat exchanger is maintained at a constant reaction temperature using an addition product of at least two of the above-mentioned substances as coolant in a sealed cooling circuit.

2. Process according to claim 1, characterised in that the coolant is maintained at a lower pressure than the reaction mixture.

3. Process according to claim 2, characterised in that the coolant is maintained at a pressure at which it evaporates in the heat exchanger.

4. Process according to claims 1 to 3, characterised in that an addition product having a boiling point above 70 degrees C (at normal pressure) is used as coolant.

5. Process according to claim 4, characterised in that isopropanol is used as coolant.

## Revendications

1. Procédé de préparation d'acide isobutyrique ou de ses dérivés par synthèse de Koch à partir de quantités à peu près stœchiométriques de propylène, de non oxyde de carbone et éventuellement d'eau ou d'un alcool dans l'acide fluorhydrique liquide servant de catalyseur de Koch, caractérisé en ce que le mélange réactionnel est maintenu à une température de réaction constante dans un échangeur de chaleur au moyen d'un produit d'addition d'au moins deux des substances mentionnées ci-dessus, servant d'agent réfrigérant parcourant un circuit fermé de refroidissement.

2. Procédé selon la revendication 1, caractérisé en ce que l'agent réfrigérant est maintenu sous une pression plus faible que le mélange réactionnel.

3. Procédé selon la revendication 2, caractérisé en ce que l'agent réfrigérant est maintenu sous une pression à laquelle il se vaporise dans l'échangeur de chaleur.

4. Procédé selon l'une quelconque des revendications 1 à 3, caractérisé en ce qu'on utilise, comme agent réfrigérant, un produit d'addition ayant un point d'ébullition supérieur à 70°C (à la pression normale).

5. Procédé selon la revendication 4, caractérisé en ce qu'on utilise de l'isopropanol comme agent réfrigérant.